# EUROPEAN PATENT SPECIFICATION

(11) **EP 3 659 634 B1**
(45) Date of publication and mention of the grant of the patent: **22.02.2023**
(21) Application number: 19275131.1
(22) Date of filing: 27.11.2019
(51) Int. Cl.: A61L 29/04, A61L 29/16, A61L 31/02, A61L 31/16

(54) **BIOACTIVE AGENT COATED MEDICAL DEVICE AND METHOD OF COATING SUCH A DEVICE**
MIT EINEM BIOAKTIVEN MITTEL BESCHICHTETE MEDIZINISCHE VORRICHTUNG UND VERFAHREN ZUR BESCHICHTUNG SOLCH EINER VORRICHTUNG
DISPOSITIF MEDICAL REVÊTU D'UN AGENT BIOACTIF ET PROCÉDÉ DE REVÊTEMENT D'UN TEL DISPOSITIF

(30) Priority: 29.11.2018 GB 201819449; 29.11.2018 US 201816203892
(43) Date of publication of application: 03.06.2020
(73) Proprietor: Cook Medical Technologies LLC, Bloomington, IN 47404 (US)
(72) Inventor: NEILAN, John, Co Galway (IE); MURRAY, David, Limerick (IE); BUTLER, James, Aherlow, Co Tipperary (IE)
(74) Representative: Williams Powell

(56) References cited:
- EP-A1- 3 192 534
- EP-A1- 3 363 478
- EP-A1- 3 431 113

## Description

### Technical Field

The present invention relates to a method of coating a medical device with a bioactive agent. The invention can be used with implantable medical devices such as stents, stent grafts, vascular filters and plugs, valvuloplasty devices and so on. It can also be applied to medical devices intended to be deployed temporarily in a patient, such as angioplasty balloons, valvuloplasty balloons, medical device deployment balloons and the like.

### Background Art

Coated medical devices, particularly endoluminally deployable medical devices, are known for a variety of medical applications. In the case of an implantable medical device, that is a device intended to be left in the patient permanently or over long periods of time, the device may coated with one or more layers of drugs intended for long term drug administration to diseased tissue. Treatment of cancers is an example. In other examples, the coating is provided in order to treat adverse body reactions caused by the medical treatment or by long term presence of a foreign object in the body, such as initial reactive hyperplasia, inflammation, thrombosis, restenosis and so on. In these cases the medical device may be deployed long term or only temporarily in a patient.

It is important that a bioactive coating on a medical device is consistent and homogeneous over the associated surface or surfaces of the device, is reliably formed from one device to another, is sufficiently well held on the device during deployment, and can be administered into the patient at the desired rate once the device is deployed. For instance, a coating on an implantable device such as a stent, filter, vascular plug or the like may need to be released over an extended period of time such as weeks, months or years; whereas a coating on a medical balloon, such as an angioplasty balloon or a device delivery balloon, may need to be released over a period of seconds or minutes.

Applying a bioactive agent to an untreated surface of a medical device often fails to form a uniform or reliable coating, leading to variable bioactive or therapeutic results. This is particularly the case with lipophilic materials including, for instance, paclitaxel, which has been proven to be a very effective anti-restenosis drug.

Attempts have been made in the art to treat one or more surfaces of medical devices to improve their biocompatibility and also to seek to improve the adherence of one or more coatings onto the medical device. These known treatments, however, have failed to provide consistent, reliable and repeatable surface characteristics for many bioactive agents. Failure to provide an adequate coating can result in failure to meet the strict drug release required by the FDA USP pharmacopeia drug device requirements and that of other regulatory bodies.

Other attempts in the art have involved providing for containment of the bioactive agent, for instance in a containment device such as a polymer matrix, by applying an outer layer or coat over the layer of bioactive material, by encapsulating the bioactive agent in capsules or other carriers, and so on. Such containment mechanisms, which restrain the bioactive material on the device and control the release of the material into the patient, can often cause other clinical issues, including reduction in the amount of bioactive material that can be carried on the medical device and inadequate release rate of the bioactive material. Furthermore, the containment mechanism can act as a target for long term restenosis and other foreign body reactions. Despite such drawbacks, containment mechanisms are still often proposed in order to seek to overcome the difficulty of adequately holding the bioactive material to the medical device and of ensuring adequate dosage of bioactive material in order to try to meet regulatory criteria.

Some examples of known surface treatments are disclosed in: "Standard Specification for Chemical Passivation Treatments for Stainless Steel Parts", Designation A967-01, ASTM International; US-8,808,272, US-4,539,061, US-2016/0355688, US-9,795,721, US-2015/0174298, EP-0,819,446, WO-2013/152713, US-7,597,924, US-7,396,582, US-6,632,470, US-8,123,799, US-9,005,960 and US-2009/171453.

### Summary of the Invention

The scope of protection is defined by the claims.

The present invention seeks to provide a method of coating a medical device with a bioactive agent.

According to an aspect of the present invention, there is provided a method of coating a medical device having a structure for implantation or disposition inside a patient as specified in claim 1.

Advantageously, the step of functionalising the at least one surface causes an increase in acidic polar components at the at least one surface.

Preferably, the method includes a further step of washing the medical device prior to the functionalisation step. This further washing step may comprise washing the at least one surface of the medical device in a solution of washing agent. The solution may be or include at least one of: sodium hydroxide, ethanol, Isopropanol (IPA), acetone, acetonitrile, hydrogen peroxide, sodium hypochlorite, a surfactant or boiling water. In one embodiment, the solution is a solution of sodium hydroxide at a ratio of 0.1 grams sodium hydroxide in 100ml water.

Advantageously, the at least one surface of the medical device solution may be bathed in solution for around 30 minutes prior to functionalisation.

The method advantageously includes the step of rinsing the at least one surface of the medical device subsequent to the pre-functionalisation washing step. The rinsing step may involve rinsing in water 10 times.

Preferably, the at least one surface of the medical device is subjected to functionalisation without drying following pre-functionalisation washing and optional rinsing.

The functionalisation preferably involves soaking the at least one surface of the medical device in a carboxylic acid. The soaking may be in a carboxylic acid solution for around 2 hours at 30°C, using preferably a 5% carboxylic acid solution.

The preferred embodiments functionalise the at least one surface by means of a citric acid solution.

Rinsing the at least one surface of the medical device in water is carried out until the acid is no longer detectable in the rinse water. The presence of acid in the rinse water may be detected by testing the pH of the water before and after rinsing. The water used for rinsing may be left to rest for a period of for example for 60 minutes, to allow its pH to settle to pH7.0 (+/- 0.1). Additionally or alternatively, an assay test may be used to determine the presence of acid in the rinse water, for example a citrate assay test may be used to test for the presence of citric acid.

Post functionalisation washing may involve: rinsing in water 5 to 20 times, preferably 10 times; and/or
rinsing in moving water for a period of 5 seconds to one hour.

It is preferred that the post-functionalisation washing step involves rinsing the at least one surface of the medical device around 10 times in water.

Preferably, the at least one surface is dried between the post-functionalisation washing step and the step of applying the bioactive agent layer.

The bioactive agent layer may:
a) consist of or be principally of bioactive material;
b) be or include a therapeutic substance;
c) be or include an anti-proliferative bioactive substance; or
d) be or include paclitaxel.

In the preferred embodiments, the bioactive agent layer is free of one or more of:
a) containment elements;
b) binding agents; and
c) time control release agents;
d) polymer or other matrix material.

The step of functionalising the at least one surface preferably does not remove or altering oxide on the at least one surface.

While citric acid is currently the preferred carboxylic acid for functionalisation, other acids have also been found to be effective, including: acetic acid, lactic acid, ascorbic acid, tannic acid, alginic acid, adipic acid, hyaluronic acid, acrylic acid, propionic acid and conjugates and derivatives thereof.

The medical device may be or include:
a) a stent or balloon;
b) a stent and wherein the structure is made of nickel titanium alloy;
c) a balloon and the coating includes an excipient.

The teachings herein also extend to a medical device having a structure as taught herein.

As disclosed in EP3192534, material improvement in surface energy and adhesion characteristics can be achieved by functionalising the surface or surfaces of the medical device with a carboxylic acid. In practice, such functionalization creates acid polar species on the surface(s), which bind by strong covalent or Lewis bonds to the conjugate bioactive material layer. Such functionalization can lead to increases in overall surface energies of around 50 Dynes/cm or more when measured by the OWRK method. This, coupled with the polar components created on the contact surface, forms a highly reactive surface to which the (bioactive) material layer binds. More particularly, the functionalisation taught herein amplifies the polar surface energy related to the type of functionalisation while suppressing other polar components of the surface energy. As described in detail below, acidification, for example, can increase the polar acid surface energy while reducing and in some cases completely suppressing the base polar components. As a result, it is not necessary to restrain the (bioactive) material in any containment mechanism, such as a containment polymer, matrix or the like.

It is preferred that the or each functionalised surface is substantially impervious to the material coating. In other words, it is preferred that the bioactive material is in the form of a distinct layer overlying the functionalised surface and preferably does not penetrate at all, or only minimally, into the functionalised surface.

Preferably, the entirety of the at least one surface is functionalised. This ensures a consistent and uniform coating of bioactive agent.

Advantageously, the coating is or includes a therapeutic substance. The coating may be or include an anti-proliferative bioactive substance, for instance paclitaxel or derivatives thereof. The coating may include one or more of a variety of other bioactive agents, of which examples are given below.

The method may also include the step of cleaning the at least one surface with an alcohol prior to functionalization, in order to remove contaminants from the surface. Advantageously, the step of cleaning the at least one surface with alcohol is carried out prior to any atomic cleaning of the surface. Ethanol is a suitable cleaning agent for this step.

The acids used for functionalisation of the surface may have a range of acidities. A strong acid, of around 1.5 pH acidity, is particularly effective.

It is preferred that the at least one acidic component includes one or more of:

O-C=O

C-O, C-OH

and

C=O.

The at least one functionalised surface may also include a dispersal facilitator, such as a C-C component.

The medical device may be of any of the varieties described above and elsewhere in this specification. Examples include stents and medical balloons. Where the medical device is a stent or has a similar support member or scaffold, the medical device may be made of a metal or metal alloy, such as a nickel titanium alloy. The stent could equally be made of other materials known in the art.

Where the medical device is or includes a balloon, or otherwise would benefit from fast release of the bioactive material, the coating may include or overlie an excipient.

Also disclosed (not part of the invention) is a medical device having a structure for implantation or disposition inside a patient, the structure including at least one coated surface, the at least one coated surface being carboxylic acid functionalised by a carboxylic acid layer of up to 2.2 nanometres in thickness and being coated with a bioactive agent layer directly on the at least one functionalised surface.

Other aspects and advantages of the teachings herein are described below in connection with the preferred embodiments disclosed herein.

### Brief Description of the Drawings

Embodiments of the present invention are described below, by way of example only, with reference to the accompanying drawings, in which:
Figure 1 is a side elevational view of an exemplary vascular stent;
Figure 2 is a schematic representation of the stent of Figure 1 in the process of being deployed in a patient's vessel to treat a stenosis;
Figure 3 is a side elevational view of an exemplary angioplasty balloon;
Figure 4 is a chart depicting the constitution of a cleaned non-functionalised contact surface of a Nitinol stent;
Figure 5 is a schematic diagram of a transverse cross-sectional view of a stent strut of the stent of Figures 1 and 2 to show the functionalised contact surface and bioactive material coating;
Figure 6 is a flow chart of the preferred stent preparation and coating method;
Figure 7 is a graph showing the coating effect of the method of Figure 6;
Figure 8 is a series of photographs showing the coating of a stent by the preferred method;
Figure 9 is a series of photographs showing a stent that has been plasma cleaned and then coated with paclitaxel; and
Figure 10 is a series of photographs of a stent that has been plasma cleaned, functionalised with citric acid and then coated with bioactive agent.

### Description of the Preferred Embodiments

It is to be understood that the drawings are schematic only and not to scale. Often only the principal components relevant to the teachings herein are shown in the drawings, for the sake of clarity.

The embodiments described below focus on a coated stent and a coated balloon. It is to be understood, however, that these are examples only and that the teachings herein can be applied to a large range of medical devices, both for temporary deployment in a patient and also for long term placement. Other examples include stent grafts, vascular filters and plugs, valvuloplasty devices, prostheses and so on.

The terms bioactive agent and bioactive material are used interchangeably in this specification and are intended to encompass materials, elements and compounds that have a therapeutic and/or preventative effect. They may, for example, be or include a therapeutic substance, such as an anti-proliferative bioactive substance of which paclitaxel is a preferred agent. Further examples of bioactive agents are given below and will also be apparent to the person skilled in the art.

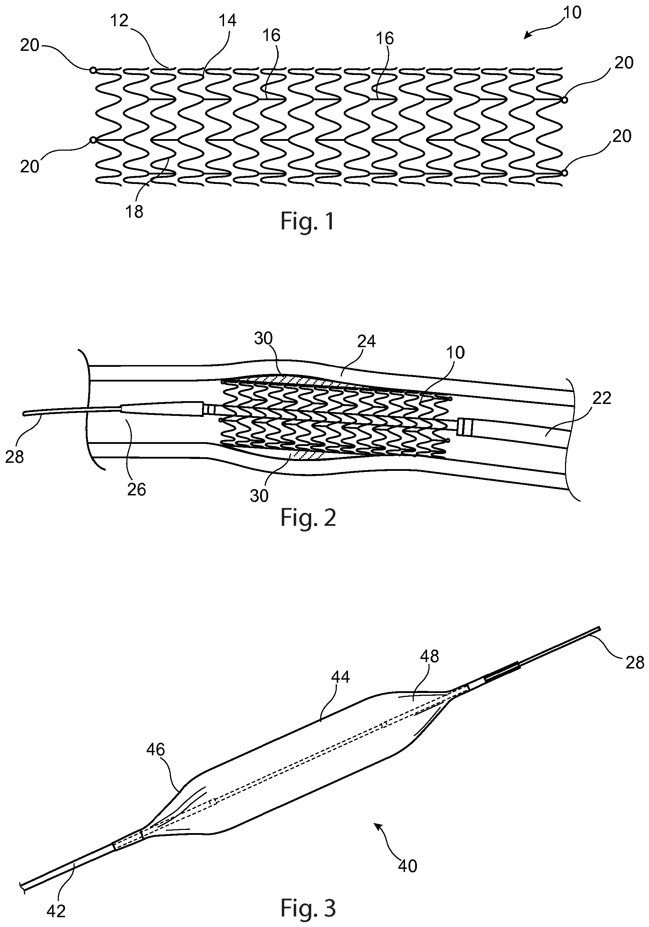

Referring first to Figure 1, there is shown an exemplary vascular stent 10 to which the teachings herein can be applied. The stent 10 is generally a tubular structure 12, in this example formed of a plurality of stent rings 14 that extend in series coaxially along the length of the tubular structure 12 and are coupled to one another by means of tie bars 16, well known in the art. In this example, the stent rings 14 are formed of a plurality of strut sections 18 arranged a zigzag shape. At the end of the stent 10 there may be provided radiopaque markers 20, again of a type well known in the art.

The stent 10 may be self-expanding or balloon expandable and made of any suitable material, of which many are known in the art.

Referring also to Figure 2, the stent 10 can be seen in the process of being deployed into a vessel 24, by means of an introducer assembly of which the distal end components 22 are visible in Figure 2. These typically include a carrier element having a dilator tip 26 at the distal end thereof. The dilator tip 26 has a lumen therein for the passage of a guide wire 28. The components of the introducer assembly are not relevant to the teachings herein.

In the example in Figure 2, the stent 10 is being deployed in order to treat a stenosis 30 of the vessel 24 and also to keep the vessel 24 open for the passage of blood therethrough.

Often, the deployment of a stent alone in the vessel does not provide a permanent solution as restenosis can often occur, closing the vessel again. This can be caused by a number of factors, including damage to the tissue of the vessel 24 during the vessel opening or angioplasty procedure, reoccurrence of the original causes of the stenosis, body reaction to the presence of a foreign body in the vessel, and so on.

Referring now to Figure 3, this shows an exemplary medical balloon 40 that may be used for angioplasty procedures, for deployment of a medical device such as a stent or stent graft, for valvuloplasty procedures or the like. The medial balloon is fitted to a balloon catheter 42 and has a substantially cylindrical balloon body 44 terminating in end cones 46, 48 that taper towards the balloon catheter 42 and fix the balloon wall to the catheter in fluid-tight manner. The balloon catheter 42 may include a lumen therein for the passage of a guide wire 28, as well as a lumen for providing inflation fluid into the balloon. The basic structure of the balloon 40 may be of a type conventional in the art, prior to modification by the teachings herein. Although Figure 3 depicts a simple balloon structure, it may have any of the features known for such balloons, including a different shape, surface roughening, texturing and so on.

An angioplasty balloon of the type depicted schematically in Figure 3 is often able to open a closed vessel in a very short period of time, for instance in seconds or minutes. While the initial vessel opening procedure is fast, there is a significant risk of future closure of the vessel, for instance by repeated collapse or restenosis. This can be caused by a number of factors including reactive hyperplasia resulting from the vessel opening procedure. Vessel closure can occur again within a few weeks or months of the medical procedure.

In the examples described briefly above in connection with Figures 2 and 3, it has been found that the administration of suitable bioactive agents into the vessel wall from the stent and/or from the medical balloon can substantially retard or prevent subsequent closure of the vessel due to restenosis. A variety of bioactive agents suitable for such purposes are known in the art including, for instance, antithrombogenic agents, thrombin inhibitors, tissue plasminogen activators, thrombolytic agents, fibrinolytic agents, vasospasm inhibitors, antiplatelet agents, antiproliferative agents and so on. A particularly effective bioactive agent known in the art is paclitaxel, other examples include dexamethasone, rapamycin, analogues of rapamycin such as Sirolimus, heparin and numerous other agents and compounds and analogues thereof. A list of suitable bioactive agents is given at the end of this specification, though it is to be understood that the list is not exhaustive.

The bioactive material is coated onto the medical device, for example the stent 10 of Figure 1 or the balloon 40 of Figure 3, so as to be released from the medical device into the tissues of the vessel 24, and should be dispensed at a rate suitable for treating the required medical condition. In the case of a stent or other implantable medical device, it may be desirable for the bioactive material to be released over a prolonged period of time, for example weeks or months. In the case of a medical device which is temporarily deployed in a patient's vessel, such as an angioplasty balloon or a device deployment balloon, the bioactive agent must typically be released from the balloon in a very short period of time, for instance within seconds or minutes, although sometimes up to an hour or more.

It is advantageous that the bioactive agent is held onto the medical device during deployment of the device in the patient without excessive loss of bioactive material into the patient's bloodstream, for instance. For this purpose, the prior art has suggested restraining the bioactive material, for instance in a containment or time release layer or matrix. Examples include: porous polymer layers into which bioactive material can be embedded, enclosed chambers holding the bioactive material, outer coatings disposed over the bioactive material and which dissolve or open during the deployment process, encapsulation of the bioactive material in micelles, capsules or pellets, and so on. Such containment measures can, however, lead to a number of disadvantages, including undesirable delayed administration of the bioactive material into body tissues, presence of a foreign substance in the body, possible onset of stenosis caused by the carrier device itself, and so on.

The inventors have found that the optimal solution is to apply the bioactive agent in the absence of any containment or time release substance and from a layer that is predominantly or entirely made of bioactive agents. In this manner, after administration of the bioactive agent or agents, the medical device remains free of agent delivery substances (polymer layers, for example) and no unnecessary carrier substances are released into the patient's body. A difficulty, however, has existed with getting the bioactive agent(s) to be held sufficiently well on the medical device.

The inventors have discovered that certain treatments of the medical device, and in particular the surface or surfaces of the device intended to be coated with one or more bioactive agents, can substantially increase the adhesion of the bioactive agent to the medical device before and during deployment of the medical device in the patient. Specifically, and as described in detail below, it is known from the prior art, and in particular EP3192534, that functionalising the surface of the medical device to be coated by way of carboxylic acidification can substantially increase the adhesive characteristics of the surface, to such an extent that it is not necessary to use other mechanisms to retain the bioactive agent on the device. They have also discovered, as demonstrated below, that this functionalisation can allow significantly more bioactive agent to be carried on the medical device.

The term functionalisation as used herein denotes the treatment of the or one or more surfaces of the medical device with a carboxylic acid to cause a change in the characteristics of the surface. Functionalisation deposits onto the surface or surfaces acid species, which bind to the device surface and provide a bonding site for the bioactive material. In many cases the carboxylic acid species is deposited as individual molecules. They do not form a polymer matrix, for instance. Bonding of the bioactive agent is by means of covalent forces, in which base/acid combinations may form a Lewis adduct. Bioactive material molecules that overlie those directly attached to their covalent species will bind to other bioactive material molecules by same species covalent bonds.

In practice, the functionalisation leads to an increase in the polar acid component of the surface or surfaces, which leads to a significant increase in the quality of adhesion of bioactive agent to the contact surface of the medical device also to a substantial improvement in uniformity of coating across the contact surface(s) of the medical device.

The functionalisation process does not remove the oxide layer on the contact surface or surfaces, but attaches carboxylic acidic components to the oxide layer. The attached carboxylic acidic components could be described as becoming part of the oxide layer. Leaving the oxide intact maintains the stability of the treated surfaces of the medical device while altering the bonding properties of the oxide layer.

Significant improvement in bioactive material retention is experienced by functionalisation alone. Better retention is achieved, though, by first cleaning the contact surface or surfaces of the medical device to remove impurities, generally acquired during and after the manufacturing process. This can substantially increase the amount of carbon functional groups on the contact surface(s) of the medical device, leading to an even more uniform coating of bioactive material across the contact surface of the medical device.

Functionalisation by acidification may be carried out by a relatively strong acid, for instance having a pH of around 1.5, although tests have shown that a large range of acids in a large pH range can be effective.

The applicant's earlier patents EP-3,192,534 and EP-3, 431,113 (corresponding to patent application EP-18275095.0) disclose methods of coating a medical device by prior functionalisation by acidification or basification of the base structure of the device. The teachings in these earlier patent applications have been found to provide good adhesion of a bioactive agent while avoiding the need for any containment mechanism to hold the bioactive agent. While these earlier methods are generally effective, the inventors have now discovered that a better and more a consistent coating or layer of bioactive agent can be applied to the medical device by a modified application procedure, described in detail below.

The examples described below relate to functionalisation by acidification. Citric acid is used as an example material for this functionalisation. Citrate could also be used, which acts as an acid as a result of its amphoteric properties. Other suitable carboxylic acids include acetic acid, lactic acid, adipic acid, alginic acid, tannic acid, oxalic acid, formic acid, levulinic acid, hyaluronic acid, acrylic acid, propionic acid, hydroxamic acid and other conjugates and derivatives. Tests have also been performed using ascorbic acid and found to be advantageous. Other carboxylic acids that may be used include: methanoic acid (formic acid), ethanoic acid (acetic acid), ethanedioc acid (oxalic acid), oxoethanoic acid (glyoxylic acid), 2-hydroxyethanoic acid (glycolic acid), propanoic acid (propionic acid, ethanecarboxylic acid), prop-2-enoic acid (acrylic acid, acroleic acid, ethylenecarboxylic acid, propene acid, vinylformic acid), propanedioic acid (malonic acid, methanedicarboxylic acid ), 2-oxopropanoic acid (pyruvic acid, α-ketopropionic acid, acetylformic acid, pyroracemic acid), 2-hydroxypropanoic acid (lactic acid, milk acid), butanoic acid (butyric acid, propanecarboxylic acid), 2-methylpropanoic acid (isobutyric acid, isobutanoic acid), butanedioic acid (succinic acid), 3-oxobutanoic acid (acetoacetic acid), (E)-butenedioic acid (fumaric acid, trans-1,2-ethylenedicarboxylic acid, 2-butenedioic acid, trans-butenedioic acid, allomaleic acid, boletic acid, donitic acid, lichenic acid), (Z)-butenedioic acid (maleic acid, cis-butenedioic acid, maleinic acid, toxilic acid), oxobutanedioic acid (oxaloacetic acid, oxalacetic acid, oxosuccinic acid), hydroxybutanedioic acid (malic acid, hydroxybutanedioic acid), 2,3-dihydroxybutanedioic acid (tartaric acid, 2,3-dihydroxysuccinic acid, threaric acid, racemic acid, uvic acid, paratartaric acid), (E)-but-2-enoic acid (crotonic acid, trans-2-butenoic acid, beta-methylacrylic acid, 3-methylacrylic acid, (E)-2-butenoic acid), pentanoic acid (valeric acid, valerianic acid, butane-1-carboxylic acid), pentanedioic acid (glutaric acid, propane-1,3-dicarboxylic acid, 1,3-propanedicarboxylic acid, n-pyrotartaric acid), 2-ketoglutaric acid (alpha-Ketoglutaric acid, 2-ketoglutaric acid, α-ketoglutaric acid, 2-oxoglutaric acid, oxoglutaric acid), hexanoic acid (caproic acid, n-caproic acid), hexanedioic acid (adipic acid, hexane-1,6-dioic acid), 2-hydroxypropane-1,2,3-tricarboxylic acid (citric acid, 3-carboxy-3-hydroxypentanedioic acid, 2-hydroxy-1,2,3-propanetricarboxylic acid), prop-1-ene-1,2,3-tricarboxylic acid (aconitic acid, achilleic acid, equisetic acid, citridinic acid, pyrocitric acid), 1-hydroxypropane-1,2,3-tricarboxylic acid (isocitric acid), (2E,4E)-hexa-2,4-dienoic acid (sorbic acid), heptanoic acid (enanthic acid, oenanthic acid, n-Heptylic acid, n-Heptoic acid), heptanedioic acid (pimelic acid), cyclohexanecarboxylic acid, benzenecarboxylic acid (benzoic acid, carboxybenzene, dracylic acid), 2-hydroxybenzoic acid (salicylic acid), octanoic acid (caprylic acid), benzene-1,2-dicarboxylic acid (phthalic acid), nonanoic acid (pelargonic acid, 1-octanecarboxylic acid), benzene-1,3,5-tricarboxylic acid (trimesic acid), (E)-3-phenylprop-2-enoic acid (cinnamic acid, trans-cinnamic acid, phenylacrylic acid, cinnamylic acid, 3-phenylacrylic acid, (E)-cinnamic acid, benzenepropenoic acid, isocinnamic acid), decanoic acid (capric acid, decanoic acid), decanedioic acid (sebacic acid 1,8-octanedicarboxylic acid), undecanoic acid (hendecanoic acid), dodecanoic acid (lauric acid, dodecylic acid, dodecoic acid, laurostearic acid, fulvic acid, 1-undecanecarboxylic acid, duodecylic acid), benzene-1,2,3,4,5,6-hexacarboxylic acid (mellitic acid, graphitic acid, benzenehexacarboxylic acid), tridecanoic acid (tridecylic acid), tetradecanoic acid (myristic acid), pentadecanoic acid (pentadecylic acid), hexadecanoic acid (palmitic acid), heptadecanoic acid (margaric acid, heptadecylic acid), octadecanoic acid (stearic acid), (9Z)-octadec-9-enoic acid (oleic acid, (9Z)-octadecenoic acid, (Z)-octadec-9-enoic acid, cis-9-octadecenoic acid, cis-Δ9-octadecenoic acid, (9Z,12Z)-octadeca-9,12-dienoic acid (linoleic acid), (9Z,12Z,15Z)-octadeca-9,12,15-trienoic acid (ALA, α-linolenic acid, cis, cis,cis-9,12,15-octadecatrienoic acid, (Z,Z,Z)-9,12,15-octadecatrienoic acid), (6Z, 9Z, 12Z)-octadeca-6, 9, 12-trienoic acid (GLA, γ-linolenic acid, gamolenic acid), (6Z,9Z,12Z,15Z)-octadeca-6,9,12,15-tetraenoic acid (SDA, stearidonic acid, moroctic acid), nonadecanoic acid (nonadecylic acid), eicosanoic acid (arachidic acid, eicosanoic acid, arachic acid), (5Z,8Z,11Z)-eicosa-5,8,11-trienoic acid (Mead acid), "(5Z,8Z,11Z, 14Z)-eicosa-5,8, 11, 14-tetraenoic acid (AA, ARA, arachidonic acid), henicosanoic acid, docosanoic acid (behenic acid), (4Z,7Z,10Z,13Z,16Z,19Z)-docosa-4,7,10,13,16,19-hexaenoic acid (DHA, cervonic acid), tricosanoic acid (tricosylic acid), tetracosanoic acid (lignoceric acid), pentacosanoic acid (pentacosylic acid), and hexacosanoic acid (cerotic acid). These acids may be used singly or in combination with one another as a mix of two or more of the above-mentioned carboxylic acids.

The skilled person will recognise from the teachings herein that a large range of acids can be used to achieve the same effects. It will be apparent that conjugates and derivatives may be equally suitable for such purposes. As an example only, hydroxamic acid is a suitable derivate of carboxylic acid.

The specific embodiments described below are directed to a stent formed of nickel titanium alloy (for instance Nitinol) which is functionalised and then coated with paclitaxel, a preferred bioactive agent, either in pure form or mixed or combined with one or more additional bioactive agents and with or without an excipient. The skilled person will appreciate that this is an example only and that the teachings herein are applicable to the other stent materials, including metals, metal alloys and also polymer based stents. The teachings herein are not limited to stents only and can be applied to other medical devices including balloons.

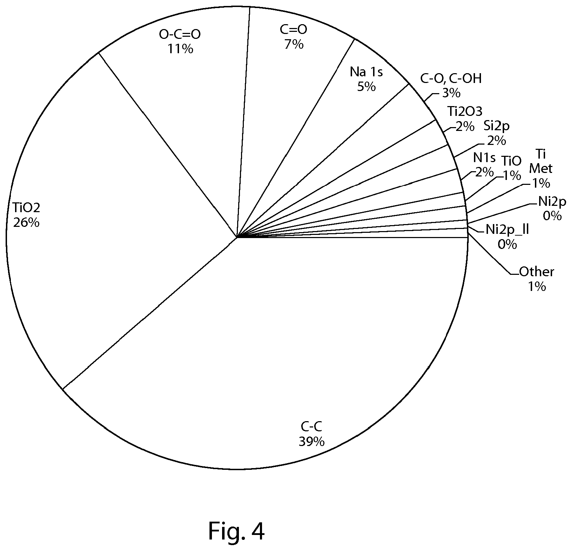

Referring now to Figure 4, this shows the constitution of a contact surface of a Nitinol stent, measured by x-ray photoelectron spectroscopy (XPS) following functionalisation of the surface with citric acid. As explained above, this functionalisation deposits onto the device surface acidic species which change the adhesive characteristics of the surface. As can be seen from Figure 4, the treated contact surface exhibits a high percentage of carbon-to-carbon (C-C) components, a high proportion of titanium dioxide and also other components including O-C=O, C=O and other carbon and oxygen components.

Functionalisation by acidification substantially reduces the amount of nickel at the contact surface, which the inventors have discovered adversely affects the retention of bioactive agents on the contact surface.

The acidic species and the titanium dioxide on the treated contact surface increase the acidic polar component of the surface energy of the contact surface of the medical device, providing good adhesion characteristics to the surface, for holding a bioactive agent (being the conjugate base) onto the contact surface of the medical device. This is substantially better than what can be achieved with a non-functionalised contact surface of a medical device. Furthermore, this process of functionalisation by acidification increases the reliability of the coating process such that a more consistent dosage of bioactive agent is applied on the contact surface during batch coating.

Even though it has been found that functionalisation by acidification only provides a notable increase in adhesion of a bioactive agent onto the medical device, it has been found that cleansing of the contact surface or surfaces of the medical device prior to acidification results in even better bioactive material retention on the medical device.

Cleaning with an alcohol such as ethanol, can remove larger impurities from the contact surface. As is described in further detail below, the preferred embodiments functionalise the contact surface or surfaces of the stent after such cleaning.

In the earlier methods disclosed in the applicant's above-mentioned patent applications, the medical device was also plasma cleaned, which creates an atomically cleaned surface, removing in particular carbon components that may have adhered to the contact surface during or after manufacture. The plasma treatment is preferably relatively low energy so as not to remove the oxide layer on the outer surface(s) of the medical device.

Suitable plasma machines include the Gatan Solarus Model 950 and Diener Femto type B. An example of an appropriate plasma cleaning treatment, for an H₂ O₂ plasma, has the following characteristics:
Vacuum = 509-531 mTorr
Turbo Pump = 750Hz, 1.0A
H₂ flow = 6.3-6.4sccm
O₂ flow = 27.4-27.5sccm
Power = 50W
Operating frequency: 13.56 MHz
Treatment time = 5 minutes.

Plasma pre-treatment results in the generation of an even greater extent of functionalised carbon bond species at the contact surface of the medical device during the process of acidification. The amount of titanium dioxide at the contact surface is substantially reduced compared to the case of functionalisation only (Figure 4). The predominant acidic species of the contact surface are, in this example: O-C=O, C-O, C-OH and C=O. These species provide an acid polar element to the surface energy of the contact surface(s) of the medical device and one which is very stable across the entire extent of the contact surface. However, plasma cleaning is not essential and in at least some embodiments, as in the preferred embodiments described below, is not carried out. It has been found, particularly for the process described herein, that the increase in surface energy produced at the treated surfaces as a result of plasma cleaning is not needed.

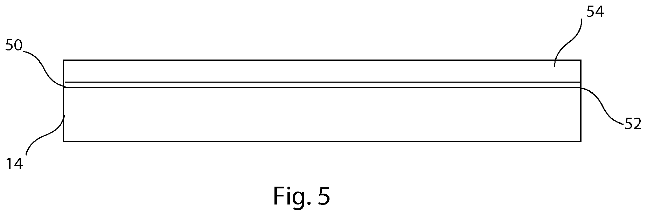

Referring briefly to Figure 5, this shows a transverse cross-sectional view of a stent, such as the stent 10 shown in Figure 1. The tubular structure 12 of the stent, in particular strut 14, thereof has had its contact surface 50 functionalised by acidification so as to form a functionalised contact surface 52 with the characteristics shown for example in Figure 4. A bioactive agent layer 54 is deposited onto the functionalised contact surface 52 (for example by spraying, rolling or dipping). It is not necessary to embed the bioactive agent in any containment matrix or layer, as is necessary with the prior art. It is preferred that the bioactive agent layer 54 is distinct from the base support (formed of the structure 14 and functionalised surface 52). Thus, the exposed surface of the bioactive agent layer 54 is solely bioactive agent or material, potentially with additional functional groups such as excipients.

There follows a description of preferred embodiments for coating a stent with a preferred bioactive material, in this case paclitaxel. The preferred embodiment functionalises at least one contact surface of the stent with citric acid applied to the contact surface(s) and thereafter a layer of paclitaxel, in the described embodiments dihydrate paclitaxel. This forms a structure with what could be described as three distinct layers as a minimum. The first layer is the base layer, being the structure of the stent, for example the Nitinol base (and it is to be understood that there will be a naturally formed oxide layer at the exposed surfaces). The second layer is a functionalisation layer of citric acid and overlying this is a third layer of dihydrate paclitaxel. The paclitaxel may be in pure form but it is not excluded that it may include other bioactive agents and in some embodiments, particularly when the device is a balloon, an excipient such as urea. The layer of bioactive material does not include any containment or time release mechanism, such as a polymer holding matrix or overlying containment device. In practice, therefore, the layer of bioactive agent constitutes the outermost layer of the device, although it is not excluded that there may be additional bioactive materials lying thereover but such that once those additional layers have been dissolved or otherwise absorbed into the patient, the layer of paclitaxel (or other bioactive agent) becomes the effective outermost layer and in a form that is not contained or otherwise restrained by any other compound or mechanism.

It has been found that in a conventional carboxylic acid deposition processes, the layer of carboxylic acid may typically be around 100 nanometres or more in thickness. Whilst such a layer can provide a good adhesion site for a variety of bioactive agents such as paclitaxel, the inventors have discovered that such an amount of carboxylic acid can be excessive and desorb in situ, causing particulate loss of bioactive material adhered to the carboxylic acid layer. The inventors have discovered that a reduction in the amount of carboxylic acid on the contact surface of the medical device does not lead to a material loss of adhesion of bioactive agent to the medical device and that only a very thin coating is required. For example, a single layer of carboxylic acid molecules need be adhered to the contact surface of the medical device and it is not necessary to have a thicker coating in which carboxylic acid molecules are bonded to one another. In tests, the inventors have discovered that forming as thin as possible a layer of carboxylic acid on the contact surface of the medical device can result in a significantly more even and complete layer of bioactive agent over the area of the contact surface or surfaces and one that holds better to the medical device when in situ.

The process described below applies carboxylic acid to the contact surface or surfaces of the medical device and then subjects the medical device (or at least the contact surface or surfaces) to a washing process to take off excess carboxylic acid. It has been found that the washing process does not completely remove the carboxylic acid from the contact surface or surfaces of the medical device but leaves a minimal layer of carboxylic acid, used for adhering a bioactive material layer thereto.

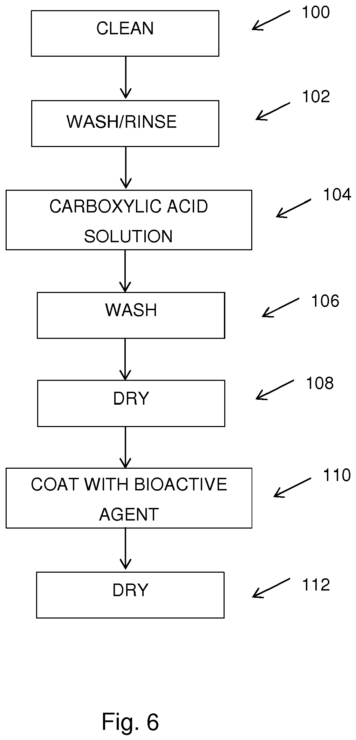

Figure 6 shows a preferred embodiment of method of coating a medical device according to the teachings herein. In this embodiment, the medical device is a nickel titanium alloy stent, functionalised with citric acid and then coated with a layer of bioactive material comprising or consisting of paclitaxel. The skilled person will appreciate that this method can readily be used or modified to coat other medical devices, including balloons and any of the other medical devices referred to herein, and that the bioactive agent can be any of those referred to herein.

In step 100 the medical device is cleaned, in this embodiment by dipping the device in a solution of sodium hydroxide (NaOH) at a concentration of 0.1 grams of NaOH in 100 ml of water. The pH of the solution with the stent added was around 12.65. The cleaning step left the stent in solution for 30 minutes or so. This cleaning/soaking stage could be shorter or longer than 30 minutes, although it has been found that 30 minutes is optimal. This stage 100 could be termed pre-functionalisation cleaning.

Following the cleaning stage 100, the stent was rinsed, at step 102, in water in order to rinse off any residual sodium hydroxide on the stent surfaces. It has been found that ten rinsing passes provide optimal rinsing. The washed and rinsed stent was then transferred immediately to a carboxylic acid solution, at step 104, in order to functionalise the surfaces of the stent. In a preferred embodiment, transfer to the carboxylic acid solution is effected without drying following rinsing.

When testing for acid in the rinse water by pH testing, the pH of the water should remain stable at approximately pH7.0 (+/-0.1) after the final rinse.

When testing for acid by an assay, in one example each acid coated stent is placed in 5ml DI water and the samples are shaken to fully mix the acid that is released from the surface, and then 20ul samples of the water is removed for use in the assay.

As indicated above, the preferred carboxylic acid used in this example is citric acid. This is preferably a 5% citric acid solution (that is 5 grams in 100 ml of water), having a pH of around 1.8. The stent was retained in the citric acid solution for around 2 hours at a temperature of around 30°C. The skilled person will appreciate that the concentration of carboxylic acid, the dipping time and the temperature may be varied and the parameters given are by way of example only.

Following the carboxylic acid functionalisation step 104, the stent was washed, at step 106, preferably in water in a process involving five to twenty, in the preferred embodiment, ten rinse passes. Rinsing can also be carried out by moving the stent in water for a period, preferably at least 5 seconds up to one hour. This may be by dipping the stent in a bath and moving or by placing the stent in a flow of water for these time periods. In the preferred embodiment, the surfaces of the stent after the final rinse were found to have in the region of pH7. This washing/rinsing step could be described as a post-functionalisation washing step.

After washing/rinsing, the stent was allowed to dry at step 108. This may be in air or in a clean environment. After drying, at step 110, the contact surface or surfaces of the stent was coated with a bioactive agent, in this example with a dihydrate paclitaxel. This coating step may be by spraying, dipping, rolling, plasma deposition or any other suitable method.

As appropriate, the stent may be dried (step 112) after coating with the bioactive agent.

While in this example the stent was cleaned with sodium hydroxide, other suitable cleaning solutions include: ethanol and mixtures of ethanol with water, isopropanol (IPA) and mixtures with water, acetone, acetonitrile, hydrogen peroxide, sodium hypochlorite, a surfactant or boiling water.

It has been found that as the carboxylic acid can be applied within wet chemical process, plasma cleaning is not necessary in the preferred embodiments.

The skilled person will appreciate that the method of Figure 6, and indeed any method in accordance with the teachings herein, could treat a plurality of stents simultaneously or sequentially in batch manner. Furthermore, the process could be applied to the entirety of the stent, so as to treat every exposed surface of the stent, or could be performed only on the surface or surfaces intended to be coated with bioactive agent, for instance the luminal or the abluminal sides of the stent.

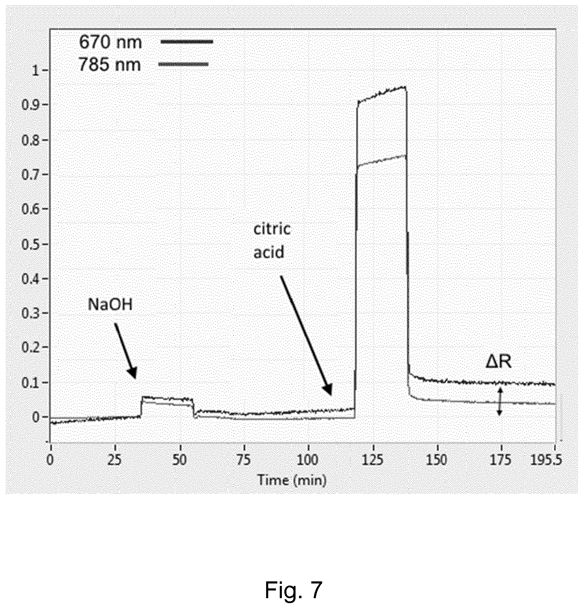

Figure 7 is a graph showing the effect of the washing and functionalisation steps of the embodiment of method shown in Figure 6. The measurements were obtained by Multi-parametric surface plasmon resonance (MP-SPR), taken at two different laser wavelengths as shown. The cleaning stage with NaOH and the functionalisation stage with citric acid were interposed with water washing stages in accordance with the described method. As can be seen, sodium hydroxide cleaning only produced a minor and temporary layer of the washed medical device surface, which was then washed off during the subsequent washing step. Coating with citric acid produced a notable layer, most but not all was removed during the subsequent water washing/rinsing stage. Even after significant water washing, there remained a coating of citric acid, depicted by the thickness measurement ΔR in the graph. Water washing removed excess citric acid very quickly, it is believed within around 5 seconds, although the washing step could be continued for a plurality of rinse cycles or for a significant time as described elsewhere in this patent specification. It has been found that the citric acid layer after washing and drying is in the region of 0.4 to 10 up to 22 nanometres, in most cases just one molecule thick. The thickness of the layer may be measured using Surface Plasmon Resonance.

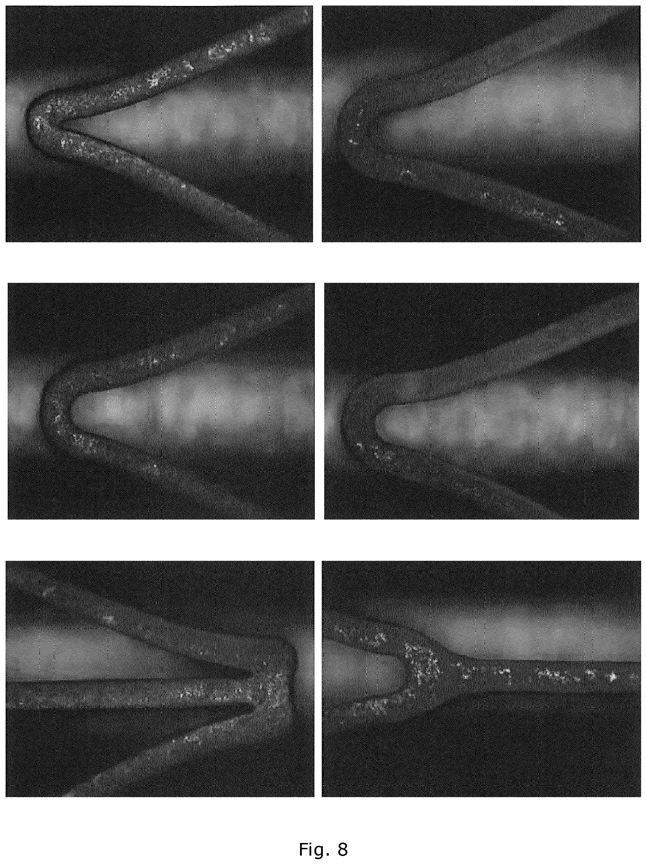

With reference now to Figure 8, this is series of photographs showing the results of coating of a stent using the method of Figure 6, particularly subjecting a stent to: (a) a pre-functionalisation wash in sodium hydroxide, (b) followed by a citric acid functionalisation, (c) followed by a washing stage to remove excess citric acid, and (d) followed by a step of coating the stent surfaces with dihydrate PTX. As can be seen at Figure 8, this process can produce a generally uniform and consistent coating of paclitaxel over the stent surfaces. In fact, the coating is notably more uniform than in the prior art.

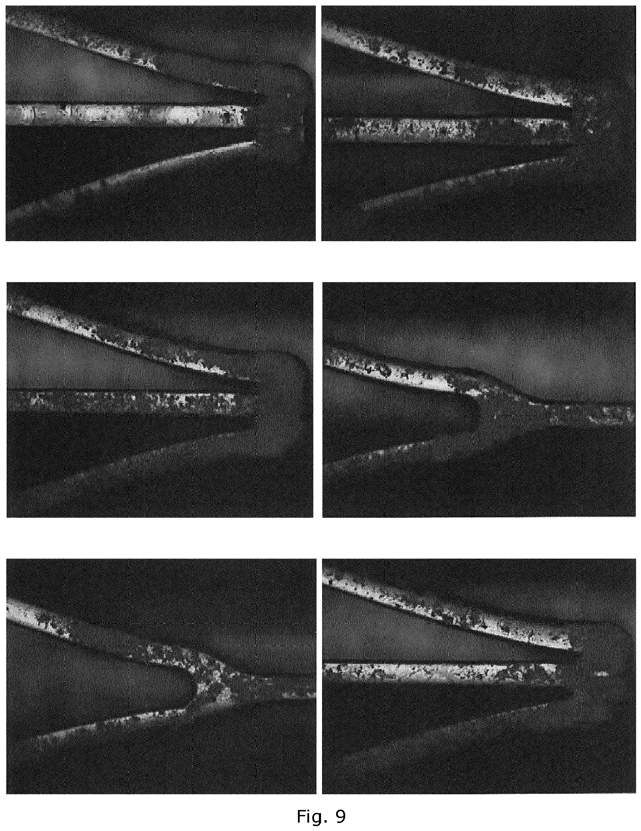

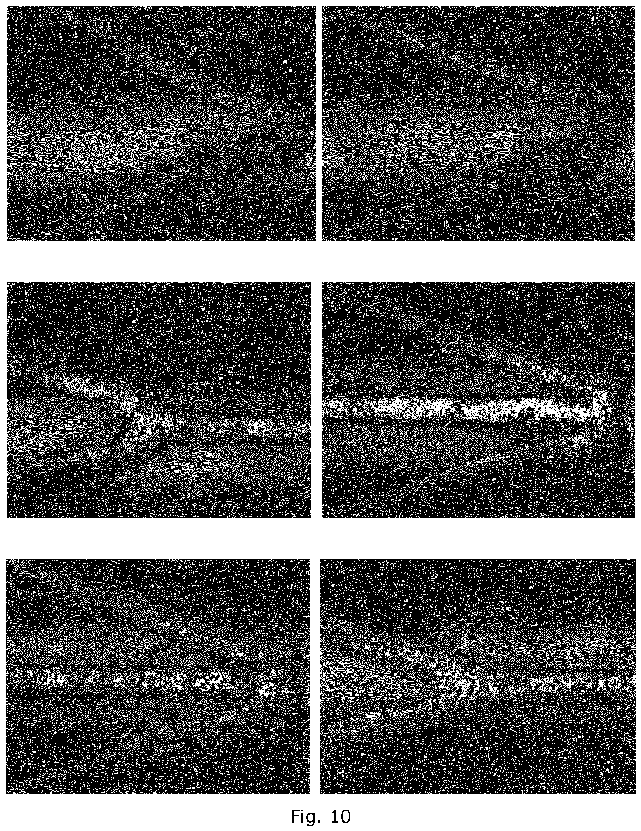

The coating of Figure 8 can be contrasted with that of Figure 9, which is a series of photographs of a stent washed in ethanol only and then having had applied thereto a coating of dihydrate paclitaxel. As can be seen in the photographs of Figure 9, the coating is incomplete and non-uniform. Similarly, referring to Figure 10, this shows a series of photographs in which a stent was coated by first plasma cleaning the stent, followed by dipping the stent in a citric acid solution, washing the stent and then applying a coating of dihydrate paclitaxel. While the coating is notably better than without the use of citric acid functionalisation, as shown in the example of Figure 8, it has been found, as described above, that if too much carboxylic acid is present, this can desorb in use, removing paclitaxel (PTX) particulate with it and resulting in a coating that is non-uniform across the contact surfaces of the stent.

Although the method and system described above and in conjunction with coating of a stent, the same method and system can be used to coat other medical devices, including medical balloons.

In the case of medical balloons and other short term use medical devices, it is generally preferred that the bioactive agent is released quickly into the patient's tissue. For this purpose, an excipient such as urea and/or urea derivatives, gallates and gallate derivatives (such as epi gallo catechin gallate), saccharides and/or saccharide derivatives, chitin and/or chitin derivatives, ascorbic acid, citric acid, sterates and/or sterate derivatives, polyvinyl pyrolidone, dicalcium phosphate dihydrate, eudragit polymers and/or eudragit polymers derivatives, cellulose and/or cellulose derivatives, PEG, poylsorbate 80, sodium lauryl sulphate, chitosan, magnesium dioxide, silicon dioxide, carbonate derivatives, plasdone, butylated hydroxyanisole, succinic acid, sodium dioctyl sulfosuccinate, precirol ATO 5, may be added to the bioactive agent layer or disposed so as to underlie the bioactive material layer. The excipient will speed up the release of the bioactive agent once the medical device is deployed within the patient, for instance by the excipient dissolving within the patient's blood plasma and providing for quick release of the bioactive agent. This can be particularly useful in treating initial reactive hyperplasia occurring as a result of angioplasty or other medical procedures. Where an excipient is used, this may be as a sublayer between the layer of bioactive material and the medical device or as a layer above the layer of bioactive material. The excipient acts to speed the release of the bioactive agent (drug for example), compared to a drug per se or a drug held in a containment or time release layer. In the case of a sublayer of excipient, the functionalisation of the surface to be coated will be matched to the nature of the excipient and the excipient matched to the bioactive agent or agents.

The bioactive material can be any of a large variety and many bioactive materials for coating medical devices are known in the art. The layer of bioactive material applied to the functionalised surfaces of the device may be of a single bioactive material or a combination of different bioactive agents, in dependence upon the desired treatment. There may also be provided other active agents in the bioactive material layer, such as excipients or other release facilitators.

The bioactive material of the coating may include at least one of: paclitaxel and/or paclitaxel derivatives, rapamycin and/or rapamycin derivatives, docetaxel and/or docetaxel derivatives, cabazitaxel and/or cabazitaxel derivatives, taxane and/or taxane derivatives, estrogen or estrogen derivatives; heparin or another thrombin inhibitor, hirudin, hirulog, argatroban, D-phenylalanyl-L-poly-L-arginyl chloromethyl ketone or another antithrombogenic agent, or mixtures thereof; urokinase, streptokinase, a tissue plasminogen activator, or another thrombolytic agent, or mixtures thereof; a fibrinolytic agent; a vasospasm inhibitor; a calcium channel blocker, a nitrate, nitric oxide, a nitric oxide promoter or another vasodilator ; an antimicrobial agent or antibiotic; aspirin, ticlopdine or another antiplatelet agent; colchicine or another antimitotic, or another microtubule inhibitor; cytochalasin or another actin inhibitor; a remodelling inhibitor; deoxyribonucleic acid, an antisense nucleotide or another agent for molecular genetic intervention; GP IIb/IIIa, GP Ib-IX or another inhibitor or surface glycoprotein receptor; methotrexate or another antimetabolite or antiproliferative agent; an anti-cancer chemotherapeutic agent; dexamethasone, dexamethasone sodium phosphate, dexamethasone acetate or another dexamethasone derivative, or another anti-inflammatory steroid; dopamine, bromocriptine mesylate, pergolide mesylate or another dopamine agonist; 60Co (having a half-life of 5.3 years), 1921r (73.8 days), 32P (14.3 days), 111In (68 hours), 10 90Y (64 hours), 99mTc (6 hours) or another radio therapeutic agent; iodine containing compounds, bariumcontaining compounds, gold, tantalum, platinum, tungsten or another heavy metal functioning as a radiopaque agent; a peptide, a protein, an enzyme, an extracellular matrix component, a cellular component or another biologic agent; captopril, enalapril or another angiotensin converting 15 enzyme (ACE) inhibitor; ascorbic acid, alphatocopherol, superoxide dismutase, deferoxyamine, a 21-aminosteroid (lasaroid) or another free radical scavenger, iron chelator or antioxidant; angiopeptin; a 14C-, 3H-, 131I1-, 32P- or 36S-radiolabelled form or other radio labelled form of any of the foregoing; or a mixture of any of these.

The teachings herein have also been tried with tannic acid with significant benefits. Tannic acid molecules are relatively large and it has been found are particularly effective for binding bioactive agents, such as paclitaxel, to a medical device, achieving significant dosages of agent to the medical device. Tannic acid can also act as an excipient as it speeds the release of the bioactive agent once hydrolysed. Other tests have used with success: lactic acid, acetic acid, formic acid, ascorbic acid, acrylic acid, propionic acid, phosphonic acid and phosphoric acid.

The teachings herein make it possible to attach bioactive agents to the surfaces of medical devices without having to rely on binding agents or polymer of other matrix materials as in the prior art. Binding agents are considered to be substances which enhance the adhesion of a bioactive material layer at the support surface and generally act to retard the release of the bioactive agent or agents. A polymer or other matrix performs a similar role. Binding agents and matrices act as containment mechanisms.

As has been described above, the teachings herein can be applied to a variety of medical devices including, in addition to the examples already indicated, vascular filters, vascular plugs, coils, neural vascular devices, pacemakers, prostheses, surgical tools, catheters, and so on. The bioactive agent can also be agents for inflammation reduction, for reducing vascular spasm, prosthesis acceptance, bone and tissue growth promoters, and so on

## Claims

1. A method of coating a medical device having a structure for implantation or disposition inside a patient, the structure including at least one surface for coating, the method including the steps of:
functionalising the at least one surface of the structure by subjecting the at least one surface to acidification by a carboxylic acid to form at least one acid functionalised surface;
washing the at least one acid functionalised surface, wherein post-functionalisation washing involves rinsing the at least one surface of the medical device in water until the rinse water remains stable at approximately pH7.0 (+/-0.1); and
applying a bioactive agent layer directly on so as to overlie the at least one functionalised and cleaned surface.

2. A method according to claim 1, including a pre-functionalisation washing step of washing the medical device prior to functionalisation; wherein the pre-functionalisation washing step optionally washes the at least one surface of the medical device in a solution of washing agent.

3. A method according to claim 2, wherein the at least one surface of the medical device is subjected to functionalisation without drying.

4. A method according to claim 2 or 3, wherein the pre-functionalisation washing step comprises
washing the at least one surface of the medical device in a solution including at least one of sodium hydroxide, ethanol, isopropanol, acetone, acetonitrile, hydrogen peroxide, sodium hypochlorite, a surfactant or boiling water.

5. A method according to claim 4, wherein the solution includes sodium hydroxide.

6. A method according to any preceding claim, wherein the functionalisation involves soaking the at least one surface of the medical device in carboxylic acid; optionally for around 2 hours at 30°C.

7. A method according to any preceding claim, wherein the functionalisation involves soaking the at least one surface of the medical device in a 5% carboxylic acid solution.

8. A method according to any preceding claim, wherein the functionalisation involves soaking the at least one surface of the medical device in a citric acid solution.

9. A method according to any preceding claim, wherein post-functionalisation washing involves :
rinsing in water 5 to 20 times, preferably 10 times; and/or
rinsing in moving water for a period of 5 seconds to one hour.

10. A method according to any preceding claim, wherein the at least one surface is dried between post-functionalisation washing and the step of applying the bioactive agent layer.

11. A method according to any preceding claim, wherein the bioactive agent layer:
a) consists of or is principally of bioactive material;
b) is or includes a therapeutic substance;
c) is or includes an anti-proliferative bioactive substance; or
d) is or includes paclitaxel.

12. A method according to any preceding claim, wherein the functionalised surface is substantially impervious to a material coating.

13. A method according to any preceding claim, wherein the at least one surface is functionalised by treatment with one or more of: citric acid, acetic acid, lactic acid, ascorbic acid, tannic acid, alginic acid, adipic acid, hyaluronic acid and conjugates and derivatives thereof.

14. A method according to any preceding claim, wherein the medical device is or includes:
a) a stent or balloon;
b) a stent and wherein the structure is made of nickel titanium alloy;
c) a balloon and the coating includes an excipient.

15. A method according to any preceding claim, wherein plasma cleaning is not carried out.

## Patentansprüche

1. Verfahren zum Beschichten einer medizinischen Vorrichtung mit einer Struktur zur Implantierung oder Anordnung in einem Patienten, wobei die Struktur mindestens eine Oberfläche zum Beschichten einschließt, wobei das Verfahren die Schritte einschließt:
Funktionalisieren der mindestens einen Oberfläche der Struktur, indem die mindestens eine Oberfläche Acidifizierung durch eine Carbonsäure unterzogen wird, um mindestens eine säurefunktionalisierte Oberfläche zu bilden;
Waschen der mindestens einen säurefunktionalisierten Oberfläche, wobei Waschen nach der Funktionalisierung Spülen der mindestens einen Oberfläche der medizinischen Vorrichtung in Wasser beinhaltet, bis das Spülwasser stabil auf einem pH-Wert von annähernd 7,0 (+/- 0,1) bleibt; und
direktes Aufbringen einer Schicht aus bioaktivem Mittel, so dass sie über der mindestens einen funktionalisierten und gereinigten Oberfläche liegt.

2. Verfahren nach Anspruch 1, das einen Präfunktionalisierungswaschschritt einschließt, bei dem die medizinische Vorrichtung vor der Funktionalisierung gewaschen wird, wobei der Präfunktionalisierungswaschschritt gegebenenfalls die mindestens eine Oberfläche der medizinischen Vorrichtung in einer Lösung von Waschmittel wäscht.

3. Verfahren nach Anspruch 2, wobei die mindestens eine Oberfläche der medizinischen Vorrichtung Funktionalisierung ohne Trocknen unterzogen wird.

4. Verfahren nach Anspruch 2 oder 3, wobei der Präfunktionalisierungswaschschritt Waschen der mindestens einen Oberfläche der medizinischen Vorrichtung in einer Lösung umfasst, die mindestens eines von Natriumhydroxid, Ethanol, Isopropanol, Aceton, Acetonitril, Wasserstoffperoxid, Natriumhypochlorit, einem Tensid oder siedendem Wasser einschließt.

5. Verfahren nach Anspruch 4, wobei die Lösung Natriumhydroxid einschließt.

6. Verfahren nach einem der vorhergehenden Ansprüche, wobei die Funktionalisierung Einweichen der mindestens einen Oberfläche der medizinischen Vorrichtung in Carbonsäure beinhaltet; gegebenenfalls für ungefähr 2 Stunden bei 30 °C.

7. Verfahren nach einem der vorhergehenden Ansprüche, wobei die Funktionalisierung Einweichen der mindestens einen Oberfläche der medizinischen Vorrichtung in 5 % Carbonsäurelösung beinhaltet.

8. Verfahren nach einem der vorhergehenden Ansprüche, wobei die Funktionalisierung Einweichen der mindestens einen Oberfläche der medizinischen Vorrichtung in einer Citronensäurelösung beinhaltet.

9. Verfahren nach einem der vorhergehenden Ansprüche, wobei Waschen nach der Funktionalisierung beinhaltet:
Spülen in Wasser für 5 bis 20 Mal, vorzugsweise 10 Mal; und/oder
Spülen in bewegtem Wasser für einen Zeitraum von 5 Sekunden bis einer Stunde.

10. Verfahren nach einem der vorhergehenden Ansprüche, wobei die mindestens eine Oberfläche zwischen dem Waschen nach der Funktionalisierung und dem Schritt des Auftragens der Schicht aus bioaktivem Mittel getrocknet wird.

11. Verfahren nach einem der vorhergehenden Ansprüche, wobei die Schicht aus bioaktivem Mittel:
a) aus bioaktivem Material besteht oder hauptsächlich aus bioaktivem Material ist;
b) eine therapeutische Substanz ist oder einschließt;
c) eine antiproliferative bioaktive Substanz ist oder einschließt; oder
d) Paclitaxel ist oder einschließt.

12. Verfahren nach einem der vorhergehenden Ansprüche, wobei die funktionalisierte Oberfläche für eine Materialbeschichtung im Wesentlichen undurchdringlich ist.

13. Verfahren nach einem der vorhergehenden Ansprüche, wobei die mindestens eine Oberfläche durch Behandlung mit einer oder mehreren von Citronensäure, Essigsäure, Milchsäure, Ascorbinsäure, Tanninsäure, Alginsäure, Adipinsäure, Hyaluronsäure und Konjugaten und Derivaten davon funktionalisiert wird.

14. Verfahren nach einem der vorhergehenden Ansprüche, wobei die medizinische Vorrichtung folgendes ist oder einschließt:
a) ein Stent oder Ballon;
b) ein Stent, und wobei die Struktur aus Nickel-Titan-Legierung gefertigt ist;
c) ein Ballon, und wobei die Beschichtung einen Hilfsstoff einschließt.

15. Verfahren nach einem der vorhergehenden Ansprüche, wobei keine Plasmareinigung durchgeführt wird.

## Revendications

1. Procédé de revêtement d'un dispositif médical possédant une structure pour une implantation ou une disposition à l'intérieur d'un patient, la structure comprenant au moins une surface pour un revêtement, le procédé comprenant les étapes de :
fonctionnalisation de l'au moins une surface de la structure en soumettant l'au moins une surface à une acidification par un acide carboxylique pour former au moins une surface fonctionnalisée par un acide ;
lavage de l'au moins une surface fonctionnalisée par un acide, un lavage post-fonctionnalisation impliquant un rinçage de l'au moins une surface du dispositif médical dans de l'eau jusqu'à ce que l'eau de rinçage reste stable à un pH d'approximativement 7,0 (± 0,1) ; et
application d'une couche d'agent bioactif directement dessus de sorte à recouvrir l'au moins une surface fonctionnalisée et nettoyée.

2. Procédé selon la revendication 1, comprenant une étape de lavage pré-fonctionnalisation de lavage du dispositif médical avant une fonctionnalisation ; l'étape de lavage pré-fonctionnalisation lavant éventuellement l'au moins une surface du dispositif médical dans une solution d'agent de lavage.

3. Procédé selon la revendication 2, l'au moins une surface du dispositif médical étant soumise à une fonctionnalisation sans séchage.

4. Procédé selon la revendication 2 ou 3, l'étape de lavage pré-fonctionnalisation comprenant un lavage de l'au moins une surface du dispositif médical dans une solution comprenant au moins l'un parmi l'hydroxyde de sodium, l'éthanol, l'isopropanol, l'acétone, l'acétonitrile, le peroxyde d'hydrogène, l'hypochlorite de sodium, un tensioactif ou de l'eau bouillante.

5. Procédé selon la revendication 4, la solution comprenant de l'hydroxyde de sodium.

6. Procédé selon une quelconque revendication précédente, la fonctionnalisation impliquant un trempage de l'au moins une surface du dispositif médical dans un acide carboxylique ; éventuellement pendant environ 2 heures à 30 °C.

7. Procédé selon une quelconque revendication précédente, la fonctionnalisation impliquant un trempage de l'au moins une surface du dispositif médical dans une solution d'acide carboxylique à 5 %.

8. Procédé selon une quelconque revendication précédente, la fonctionnalisation impliquant un trempage de l'au moins une surface du dispositif médical dans une solution d'acide citrique.

9. Procédé selon une quelconque revendication précédente, un lavage de post-fonctionnalisation impliquant :
un rinçage dans de l'eau 5 à 20 fois, préférablement 10 fois ; et/ou
un rinçage dans de l'eau se déplaçant, pendant une période de 5 secondes à une heure.

10. Procédé selon une quelconque revendication précédente, l'au moins une surface étant séchée entre un lavage de post-fonctionnalisation et l'étape d'application de la couche d'agent bioactif.

11. Procédé selon une quelconque revendication précédente, la couche d'agent bioactif :
a) étant constituée de ou étant principalement de matière bioactive ;
b) étant ou comprenant une substance thérapeutique ;
c) étant ou comprenant une substance bioactive antiproliférative ; ou
d) étant ou comprenant le paclitaxel.

12. Procédé selon une quelconque revendication précédente, la surface fonctionnalisée étant sensiblement imperméable à un revêtement de matériau.

13. Procédé selon une quelconque revendication précédente, l'au moins une surface étant fonctionnalisée par traitement avec l'un ou plusieurs parmi : l'acide citrique, l'acide acétique, l'acide lactique, l'acide ascorbique, l'acide tannique, l'acide alginique, l'acide adipique, l'acide hyaluronique et des conjugués et des dérivés correspondants.

14. Procédé selon une quelconque revendication précédente, le dispositif médical étant ou comprenant :
a) un stent ou un ballon ;
b) un stent et la structure étant composée d'un alliage de nickel et de titane ;
c) un ballon et le revêtement comprenant un excipient.

15. Procédé selon une quelconque revendication précédente, un nettoyage au plasma n'étant pas mis en œuvre.
